(19)

**Europäisches Patentamt**
**European Patent Office**
**Office européen des brevets**

(11) **EP 1 779 854 A1**

(12) **EUROPEAN PATENT APPLICATION**
published in accordance with Art. 158(3) EPC

(43) Date of publication:
**02.05.2007 Bulletin 2007/18**

(21) Application number: **05755167.3**

(22) Date of filing: **29.06.2005**

(51) Int Cl.:
*A61K 31/704* (2006.01)　　*A61K 9/02* (2006.01)
*A61K 47/02* (2006.01)　　*A61K 47/14* (2006.01)
*A61K 47/18* (2006.01)　　*A61K 47/26* (2006.01)
*A61K 47/34* (2006.01)　　*A61K 47/44* (2006.01)
*A61P 1/06* (2006.01)　　*A61P 17/00* (2006.01)
*A61P 37/08* (2006.01)

(86) International application number:
**PCT/JP2005/011958**

(87) International publication number:
**WO 2006/001477 (05.01.2006 Gazette 2006/01)**

(84) Designated Contracting States:
**AT BE BG CH CY CZ DE DK EE ES FI FR GB GR HU IE IS IT LI LT LU MC NL PL PT RO SE SI SK TR**

(30) Priority: **29.06.2004 JP 2004191275**
**22.12.2004 JP 2004371222**

(71) Applicant: **Minophagen Pharmaceutical Co., Ltd.**
**Tokyo, 107-0052 (JP)**

(72) Inventors:
• **HANADA, Takashi,**
  **MINOPHAGEN PHARMACEUTICAL CO. Ltd**
  **Zama-shi,**
  **Kanagawa 2280002 (JP)**
• **ISHIGURO, Akiko,**
  **MINOPHAGEN PHARMACEUTICAL CO. Ltd**
  **Zama-shi,**
  **Kanagawa 2280002 (JP)**

• **HAMADA, Yuji,**
  **MINOPHAGEN PHARMACEUTICAL CO., Ltd.**
  **Zama-shi,**
  **Kanagawa 2280002 (JP)**
• **INOUE, Hideo,**
  **MINOPHAGEN PHARMACEUTICAL CO., Ltd.**
  **Zama-shi,**
  **Kanagawa 2280002 (JP)**
• **SATO, Toshitsugu**
  **Zama-shi,**
  **Kanagawa 2280002 (JP)**

(74) Representative: **Snodin, Michael D. et al**
  **Eric Potter Clarkson LLP**
  **Park View House**
  **58 The Ropewalk**
  **Nottingham NG1 5DD (GB)**

(54) **GLYCYRRHIZIN-CONTAINING SUPPOSITORY COMPOSITIONS FOR RECTAL INFUSION**

(57)　The present invention provides a glycyrrhizin-containing suppository composition for rectal infusion which contains: at least one selected from glycyrrhizin and pharmaceutically acceptable salts thereof; a nonionic surfactant; an oily base; and water, the glycyrrhizin-containing suppository composition for rectal infusion having a (solid-liquid) phase transition point of 10 to 4500 Pa at 37°C. Also, the present invention provides a glycyrrhizin-containing suppository composition for rectal infusion which contains: at least one selected from glycyrrhizin and pharmaceutically acceptable salts thereof; an oily base; water; and an alkali.

EP 1 779 854 A1

**Description**

TECHNICAL FIELD

The present invention relates to a glycyrrhizin-containing suppository composition for rectal infusion, a production method thereof, and a hepatic disease-therapeutic agent, skin disease-therapeutic agent, antiallergic agent, SARS therapeutic agent, and AIDS therapeutic agent containing the glycyrrhizin-containing suppository composition for rectal infusion.
The present invention claims priority on Japanese Patent Application No. 2004-191275 filed on June 29, 2004, and Japanese Patent Application No. 2004-371222 filed on December 22, 2004, the contents of which are incorporated herein by reference.

BACKGROUND ART

It has been known that glycyrrhizin (hereinafter, abbreviated as GL) and salts thereof exhibit wide bioactivity such as antiallergic activity, anti-inflammatory activity, immunomodulatory activity, hepatocellular damage inhibitory activity, hepatocellular proliferation promoter activity, virustatic activity, or the like, so they have been used as clinical medicines since a long time ago. Currently, they are widely used alone or as a combination agent with an amino acid, for treating hepatic disease, skin disease, various allergies, inflammation, severe acute respiratory syndrome (SARS), acquired immune deficiency syndrome (AIDS), or the like (see, for example, Nonpatent Documents 1 and 2).

[Nonpatent Document 1] THE LANCET, Vol. 361, No. 934, Pages 2045-2046, 2003
[Nonpatent Document 2] Antiviral Research, 11 (1989), 255-262

In particular, there is much use as a hepatic disease - therapeutic agent applied for viral chronic hepatic disease. In the current treatment of viral chronic hepatic disease, a radical cure using interferon (hereinafter, abbreviated as IFN) is being established with the aim of completely eliminating viruses, but approximately half the cases do not attain a radical cure. Accordingly, a liver supporting therapy for stabilizing transaminase using a GL preparation has been required for a long time so as to suppress the progress of hepatic inflammation or hepatic cirrhosis and further inhibit hepatic tumorigenesis or the like.
As an administration route of a GL preparation, there are generally two routes, that is, intravenous administration and oral administration. In the case of the oral administration, effects of GL cannot be sufficiently exhibited, so GL is usually administered intravenously using an injectable product in actual clinical practice. The reason for this is that GL orally administered exists in an anionic form dissociated at around neutral pH of the intestines, so is poorly absorbed in the intestines, and is decomposed to glycyrrhetinic acid (hereinafter, abbreviated as GA), which is an aglycon, by intestinal bacteria or the like, in the gastrointestinal tract, and so almost no GL is incorporated in the body.
However, the intravenous administration is highly invasive, for example, it injures the blood vessels or the like, and so the intravenous administration heavily burdens the patient. In the case of chronic hepatic disease, since the treatment thereof extends over a long period of time, there is a problem in which the Q.O.L (quality of life) of the patient is significantly decreased, in particular.
In view of the above, a rectal suppository has attracted the attention. For example, Patent Document 1 discloses a suppository composition in which GL is contained in an oily base. Also, GL suppository compositions containing a conventional oily base have been used as hospital preparations (see, for example, Nonpatent Document 3). Since suppositories containing these suppository compositions exhibit no invasiveness, the Q.O.L of the patient can be significantly improved.

[Patent Document 1] Japanese Unexamined Patent Application, First Publication No. H 3-2122.
[Nonpatent Document 3] Hepatol. Res. 26 (2003), 10 to 14

However, these suppository compositions have a problem in which the absorbability of the GL is low and effects thereof are not sufficiently exhibited. Accordingly, it is required that the content of the GL in these suppository compositions and the dose administered per day be more than those of usual ones. For example, in the case of the above-mentioned hospital preparations, a suppository containing 300 mg of GL is administered twice per day, as a result of which the GL is administered at a dose significantly exceeding 200 mg, this value being considered as the daily maximum dose of the GL in actual clinical practice. Such massive GL-administration causes various side-effects or other safety probems, if administered for a long time in particular.
Since the GL exhibits such high gelation effects that GL is used as a gelatinizing agent, the GL is hardened by mixing it with an oily base and water. Accordingly, when these are mixed using an apparatus such as a homomixer, emulsifier, or the like, it is difficult to uniformly mix them, and time and cost are required, so industrial production is difficult.

In contrast, oral preparations using an absorption promoter such as capric acid or the like for enhancing the absorption of GL have been proposed (see, for example, Patent Documents 2 and 3).

[Patent Document 2] Japanese Unexamined Patent Application, First Publication No. H 10-226650.
[Patent Document 3] Japanese Unexamined Patent Application, First Publication No. H 6-192107.

However, these oral preparations are required to contain, as an absorption promoter, a medium chain fatty acid such as capric acid exhibiting strong mucosal irritation or the like, and so have safety problems in a similar manner to the above. Also, as described above, almost all GL is decomposed to GA in the gastrointestinal tract, so effects thereof are not sufficiently exhibited.

DISCLOSURE OF THE INVENTION

[Problems to be Solved by the Invention]

The present invention has been achieved so as to solve the above-mentioned problems, and has as its object to provide a GL-containing suppository composition for rectal infusion that can exhibit sufficient effects at a dose less than that of prior arts and can be industrially manufactured with ease, a production method thereof, and a hepatic disease-therapeutic agent, skin disease-therapeutic agent, antiallergic agent, SARS therapeutic agent, and AIDS therapeutic agent, containing the GL-containing suppository composition for rectal infusion.

[Means for Solving the Problems]

As a result of earnest investigation, the inventors of the present invention found that there is a correlation between the phase transition point of a composition containing a GL and/or salts thereof, an oily base, a nonionic surfactant, and water at 37°C and the rectal absorption of the GL, and found that the rectal absorption is enhanced by adjusting the phase transition point within a particular range.

Also, as a result of earnest investigation, the inventors of the present invention found that when a GL and/or salts thereof is mixed with an oily base, water, and an alkali, the mixture is softened and thereby industrial production can be easily conducted.

That is, a first aspect of the present invention provides a GL-containing suppository composition for rectal infusion containing: at least one selected from a GL and pharmaceutically acceptable salts thereof; a nonionic surfactant; an oily base; and water, in which the (solid-liquid) phase transition point thereof at 37°C is within a range from 10 to 4500 Pa.

Moreover, a second aspect of the present invention provides a GL-containing suppository composition for rectal infusion containing: at least one selected from a GL and pharmaceutically acceptable salts thereof; an oily base; water; and an alkali.

The alkali may be at least one selected from the group consisting of alkali metal phosphates, alkali metal carbonates, alkali metal hydroxides, alkaline earth metal hydroxides, amines, and an ammonia.

Moreover, the second aspect of the GL-containing suppository composition for rectal infusion may further contain a nonionic surfactant, and the nonionic surfactant may be at least one selected from the group consisting of sucrose fatty acid esters and polyglycerol fatty acid esters.

Moreover, the present invention provides a hepatic disease-therapeutic agent containing the GL-containing suppository composition for rectal infusion.
Also, the present invention provides a skin disease-therapeutic agent containing the GL-containing suppository composition for rectal infusion.

Moreover, the present invention provides an antiallergic agent containing the GL-containing suppository composition for rectal infusion.

Also, the present invention provides a SARS therapeutic agent containing the GL-containing suppository composition for rectal infusion.
Also, the present invention provides an AIDS therapeutic agent containing the GL-containing suppository composition for rectal infusion.

Moreover, the present invention provides a method for producing the GL-containing suppository composition for rectal infusion.

[Effects of the Invention]

The GL-containing suppository composition for rectal infusion according to the present invention can exhibit an excellent GL-absorbability and a high medicinal effects by rectal administration. Accordingly, the content of the GL contained in the GL-containing suppository composition for rectal infusion can be made lower than that of conventional preparations, and thereby exhibits high safety.

The GL-containing suppository composition for rectal infusion according to the present invention can contain the GL at a high concentration without impairing the absorbability thereof. Accordingly, in the case of a preparation containing 10% of the GL, by administrating 2 g of the preparation, the GL can be administered at the maximum daily dose, 200 mg, of the GL, which is practical from the standpoint of clinical use, and can exhibit therapeutic effects comparable with intravenous administration.

Also, since the suppository composition for rectal infusion according to the present invention is soft, the suppository composition can be industrially manufactured with ease. Since the composition is soft even after it is made into a preparation, the composition can be easily discharged from a container, and easily used.

Thus, the GL-containing suppository composition for rectal infusion according to the present invention can be used easily, and is not invasive, so the Q.O.L of the patient can be significantly improved.

Moreover, the GL-containing suppository composition for rectal infusion is not required to contain an absorption promoter which may stimulate the mucous membrane, such as a capric acid or the like, and thereby, exhibits high safety.

Since the GL-containing suppository composition for rectal infusion according to the present invention can be industrially produced and used with ease, the suppository composition can be applied for improving the same diseases as those currently treated with GL-containing injectable products in clinical practice, such as, for example, hepatic dysfunction caused by acute or chronic hepatic disease, skin disease such as eczema, skin inflammation, urticarial rash, universal pruritus, drug eruption, toxic eruption, or the like, various allergies, SARS, AIDS, or the like.

In particular, the GL-containing suppository composition for rectal infusion according to the present invention can be preferably used as a hepatic disease-therapeutic agent, skin disease-therapeutic agent, antiallergic agent, SARS therapeutic agent, or AIDS therapeutic agent. Among these, the suppository composition can be more preferably used as the hepatic disease-therapeutic agent, and can be used very conveniently in comparison with conventional injectable products for the patient with chronic hepatic disease requiring long-term treatment, in particular.

The GL-containing suppository composition for rectal infusion according to the present invention can provide a treatment with extremely low cost in comparison with IFN treatment, and thereby, is useful from the standpoint of medical economics.

BRIEF DESCRIPTION OF THE DRAWINGS

FIG. 1 is a graph showing results of Test Example 4.
FIG. 2 is a graph showing results of Test Example 7.

BEST MODE FOR CARRYING OUT THE INVENTION

In the following, the preferable aspects of the present invention will be explained. However, the present invention is not limited to the following aspects.

A first aspect of the GL-containing suppository composition for rectal infusion according to the present invention contains: at least one selected from a GL and pharmaceutically acceptable salts thereof; a nonionic surfactant; an oily base; and water, and the (solid-liquid) phase transition point thereof at 37°C is within a range from 10 to 4500 Pa.

When the (solid-liquid) phase transition point at 37°C is within the above-mentioned range, the rectal absorption of the GL is enhanced, and so the composition can exhibit sufficient effects even if its dose is less than that of conventional preparations. One of the reasons for increasing the rectal absorption of the GL may be that the dispersibility of the GL-containing suppository composition for rectal infusion in the rectum is suitable for absorbing the GL from the rectum when the phase transition point is within the above-mentioned range. That is, it is supposed that a conventional GL suppository composition exhibits a low GL-absorbability because the GL exhibits such a high gelation effect that the composition is gelatinized by a slight moisture content secreted in the rectum and so the dispersibility thereof in the rectum deteriorates. In contrast, since the GL-containing suppository composition for rectal infusion of the present aspect has the (solid-liquid) phase transition point within the particular range, the dispersibility thereof in the rectum is improved, and thereby, the rectal absorption of the GL is enhanced.

In the present aspect, the (solid-liquid) phase transition point is preferably 10 to 3000 Pa, more preferably 10 to 2000 Pa, and even more preferably 15 to 1500 Pa.

The phrase "(solid-liquid) phase transition point at 37°C" means shear stress (Pa) at the phase transition from liquid phase to solid phase or solid phase to liquid phase of the GL-containing suppository composition for rectal infusion at 37°C.

The (solid-liquid) phase transition point can be determined as follows, for example.

First, the viscoelasticity (G' (storage elastic modulus) and G" (loss elastic modulus)) of the GL-containing suppository composition for rectal infusion is measured, assuming an internal body temperature of 37°C, in an oscillation stress sweep measurement mode using an apparatus for measuring viscoelasticity (for example, RheoStress RS 600 manufactured by Thermo Electron K.K. (Karlsruhe)) with a parallel plate (20φ, 35φ, 60φ) or a cone plate (60φ, 1°), suitably selected as a measurement geometry.

Next, the viscoelasticity (Pa) is plotted on a left longitudinal axis, and the shear stress τ (Pa) is plotted on a horizontal axis, to make graphs of the following formulae: G'=f (τ) and G"=g (τ). Also, phase angles δ(°) are plotted on a right longitudinal axis, to make a graph.

The shear stress τ (Pa) at the intersection of G' and G", that is, the shear stress τ (Pa) at the phase angle δ of 45° (that is, tangent δ of 1) is determined as the (solid-liquid) phase transition point.

The (solid-liquid) phase transition point of the GL-containing suppository composition for rectal infusion can be lowered by increasing the content of the nonionic surfactant, adding an alkali or a pH adjuster, or the like. Also, the phase transition point can be adjusted by adjusting HLB or kinds of the nonionic surfactant used or water content.

As the GL and pharmaceutically acceptable salts thereof used in the present aspect, those available from Minophagen Pharmaceutical Co., LTD., can be exemplified. As the pharmaceutically acceptable salts thereof, ammonium salts such as GL ammonium salt or the like; sodium salts such as GL monosodium salt, GL disodium salt, or the like; potassium salts such as GL monopotassium salt, GL dipotassium salt, or the like, can be exemplified. In addition to these, calcium salts, magnesium salts, aluminum salts, various organic amine salts of the GL, and the like can be exemplified. These may be used alone or in combination with at least two kinds thereof.

The GL-containing suppository composition for rectal infusion of the present aspect contains at least one selected from the GL and the pharmaceutically acceptable salts thereof (hereinafter, these may be collectively abbreviated as GL (salts)) in an amount of preferably 30% by mass or less, more preferably 20% by mass or less, even more preferably 1 to 20% by mass, further even more preferably 1 to 15% by mass, and particularly preferably 5 to 15% by mass. When the content of the GL (salts) exceeds 30% by mass, it may become difficult to ensure the uniform dispersion of the GL (salts) in the GL-containing suppository composition for rectal infusion, and the absorbability of the GL (salts) may deteriorate. When the content of the GL (salts) is 1% by mass or more, the effects of the GL (salts) can be sufficiently exhibited. When the content is within the above-mentioned range, in the case of a preparation containing 10% by mass of the GL (salts), for example, the GL can be administered at the daily maximum dose of 200 mg by administrating 2g of the preparation, and thus, the GL-containing rectal infusion suppository can be practically used from the standpoint of the clinical practice.

As the nonionic surfactant used in the present aspect, sugar esters such as polyglycerol fatty acid esters, glyceryl fatty acid esters, sorbitan fatty acid esters (C12 to 18), polyoxyethylene (POE) sorbitan fatty acid esters (C12 to 18), sucrose fatty acid esters, or the like, fatty acid esters such as POE fatty acid esters (C12 to 18), POE resin acid esters, POE fatty acid diesters (C12 to 18), or the like; alcohols such as POE alkyl ethers (C12 to 18); alkylphenols such as POE alkyl (C8 to 12) phenyl ethers, POE dialkyl (C8 to 12) phenyl ether, POE alkyl (C8 to 12) phenyl ether formalin condensates, or the like; POE - POP block polymers such as POE - polyoxypropylene (POP) block polymers, alkyl (C12 to 18) POE - POP block polymer ethers, or the like; alkylamines such as POE alkylamine (C12 to 18), POE fatty acid amides (C12 to 18), or the like; vegetable oils such as POE castor oils, POE hardened castor oils, or the like can be exemplified.

Among these, at least one selected from the group consisting of sucrose fatty acid esters and polyglycerol fatty acid esters is preferable.

As the sucrose fatty acid esters, those of which the fatty acid ester has 12 to 18 carbon atoms, such as a laurate ester, palmitate ester, stearic acid ester, or the like and of which the ester content contains 55% or more of a monoester can be exemplified. Commercially available examples thereof include those manufactured by Mitsubishi-Kagaku Foods Corporation under the trade name of "J-1811 ", "J-1816", "J-1616", and "J-1216", those manufactured by DAI-ICHI KOGYO SEIYAKU CO., LTD., under the trade name of "SS", "F-160", "F-110", "F-20W", "F-10", and the like.

Commercially available examples of the polyglycerol fatty acid esters include those manufactured by Mitsubishi-Kagaku Foods Corporation under the trade name of "S-24D", "L-7D", PGMSV manufactured by The Nisshin OilliO Group, Ltd., and the like.

It is preferable that the nonionic surfactant have a HLB (hydrophilic-lipophilic balance) from 8 to 19, and more preferably from 10 to 19. When the HLB is within the above-mentioned range, the absorbability of the GL is particularly preferable. As the nonionic surfactant, one kind thereof may be used alone, or at least two kinds thereof may be used in combination. When at least two kinds of the nonionic surfactant are used, at least two kinds with different HLB values are preferably used, each HLB value may be 1 to 19, and a nonionic surfactant with a high HLB value and a nonionic surfactant with a low HLB value can be used in combination.

The content of the nonionic surfactant in the GL-containing suppository composition for rectal infusion of the present aspect is preferably 0.5 to 20% by mass, and more preferably 1 to 15% by mass. When the content of the nonionic surfactant is within the above-mentioned range, the emulsifiability and dispersibility of the GL in the rectum are improved, and the absorption of the GL (salts) from the rectum is significantly increased, so high medicinal effects can be exhibited.

The oily base used in the present aspect is not particularly limited, and those generally used for a suppository can be used. Examples thereof include medium chain fatty acid triglycerides, plant-derived fatty acid glyceryl esters such as soybean oils, olive oils, and the like.

Examples of the medium chain fatty acid triglycerides include those containing fatty acids having 6 to 12 carbon atoms, such as caproic acid, caprylic acid, capric acid, dodecanoic acid, and the like. Three medium chain fatty acids contained in the medium chain fatty acid triglyceride may be identical to or different from each other.

As the medium chain fatty acid triglyceride, those of which fatty acid components contain 50 to 80% by mass of caprylic acid and 20 to 45% by mass of capric acid are particularly preferable. Examples of such medium chain fatty acid triglycerides include a commercially available one manufactured by SASOL CHEMICAL INDUSTRIES LTD., under the trade name of "MIGLYOL 812".

The content of the oily base in the GL-containing suppository composition for rectal infusion of the present aspect is preferably 10 to 50% by mass, and more preferably 10 to 40% by mass. When the content of the oily base is within the above-mentioned range, the stability of the preparation is high, and effects of the present aspect can be sufficiently exhibited.

As water used in the present aspect, purified water or the like can be exemplified.
The content of water in the GL-containing suppository composition for rectal infusion of the present aspect is preferably 20 to 70% by mass, and more preferably 30 to 60% by mass. When the content of water is outside the above-mentioned range, it may become difficult to ensure the uniform dispersion of the GL in the GL-containing suppository composition for rectal infusion, and thereby the absorption of the GL (salts) may deteriorate.

In addition to the above, the GL-containing suppository composition for rectal infusion of the present aspect may contain optional components without impairing the effects of the present aspect. Examples of the optional components include antiseptics, various stabilizers, and the like.

Also, the GL-containing suppository composition for rectal infusion of the present aspect may contain an alkali or a pH adjuster. By formulating the pH adjuster, the phase transition point of the GL-containing suppository composition for rectal infusion can be controlled.

The GL-containing suppository composition for rectal infusion of the present aspect can be obtained as a semisolid composition by mixing and dissolving the GL (salts), the nonionic surfactant, and the oily base, and then adding and mixing water to the solution, for example. The resultant is filled in a container such as a syringe, tube, or the like, so as to use it as an infusion-type suppository.

Mixing can be conducted, for example, using a homogenizer, automatic mortar, or the like, in accordance with a method generally used for preparing an emulsion.

The absorption of the GL (salts) contained in the GL-containing suppository composition for rectal infusion of the present aspect can be evaluated by measuring the amount of the GL (salts) excreted in the bile and calculating the ratio thereof with respect to the amount of the administered GL (salts), that is, collection rate (%) in the bile.

As shown in Nonpatent Document 4, when the absorption of the GL (salts) from the intestine and the excretion clearance thereof in the liver are measured, the absorption of the GL (salts) and the excretion clearance thereof are almost the same. Accordingly, it is supposed that the GL (salts) absorbed from the intestine is carried to the liver through the portal vein, and influenced by the strong first pass effect at the liver, as a result of which almost all of the GL (salts) is excreted into the bile without modification and a portion thereof enters the systemic circulation. Also, most (80 to 90% in the case of the rat (see Nonpatent Document 5) and 80% in the case of the human (see Nonpatent Document 6)) of the GL (salts) injected intravenously into the body is excreted into the bile.
Indeed, as a result of examination carried out by the inventors of the present invention, 94.8% of the GL (salts) injected jugularly to the rat had been excreted into the bile for 6 hours. Accordingly, it is supposed that almost all of the GL (salts) absorbed from the gastrointestinal tract is excreted from the liver into the bile and a portion thereof enters the systemic circulation. Accordingly, the method for evaluating the absorbability by the collection rate of the GL in the bile, the GL in the bile being considered to be passed through the liver, is considered to be suitable.

[Nonpatent Document 4] Chem. Pharm. Bull. 38(1), p212 to 218, (1990)
[Nonpatent Document 5] J. Pharm. Sci., 75(7), p672 to 675 (1986)
[Nonpatent Document 6] Pharmaceutical Society of Japan, 111 annual abstract, 28M, 11-3

Specifically, the absorbability is evaluated by collecting the bile in accordance with a conventional method as each sample, determining the quantity of the GL (salts) in the sample by a semimicro HPLC method, and then calculating the collection rate (%) in the bile from the quantity of the GL (salts) in the bile in accordance with the following formula.

Collection rate (%) in the bile = [the quantity of the GL (salts) collected in the bile] × 100

/ [the quantity of the GL (salts) administered]

As described above, the GL-containing suppository composition for rectal infusion of the present aspect exhibits high absorbability of GL (salts), and so can exhibit sufficient effects even if the dose thereof is less than that of the prior parts. Accordingly, the suppository composition can be applied for the same diseases as those treated with GL-containing injectable products currently used in clinical practice, such as hepatic dysfunction caused by acute or chronic hepatic disease, skin disease such as eczema, skin inflammation, urticarial rash, universal pruritus, drug eruption, toxic eruption, or the like, various allergies, SARS, AIDS, or the like. Among these, the suppository composition is preferably used in a hepatic disease-therapeutic agent and particularly applied for patients with chronic hepatic disease requiring long-term treatment with great convenience with respect to conventional injectable products.

The hepatic disease-therapeutic agent, skin disease-therapeutic agent, antiallergic agent, SARS therapeutic agent, and AIDS therapeutic agent, containing the GL-containing suppository composition for rectal infusion are suitably prescribed by a clinician in accordance with the body weight or health condition of the patient to be treated. The agent may be administered once or several times per day, for example, up to a GL-ceiling of 200 mg.

As described above, by adding the nonionic surfactant to the GL (salts), oily base, and water, the GL-containing suppository composition for rectal infusion of the present aspect can exhibit improved absorbability of the GL (salts), and thereby, can exhibit sufficient effects even if the dose thereof is less than that of the prior arts. One of the reasons for this is supposed to be that the dispersibility of the GL-containing suppository composition for rectal infusion in the rectum is improved by adjusting the phase transition point within the particular range.

The GL-containing suppository composition for rectal infusion of the present aspect need not contain an absorption promoter that has a strong biological stimulus property, such as capric acid, and thereby is excellent in the safety.

A second aspect of the GL-containing suppository composition for rectal infusion according to the present invention is characterized by containing: at least one selected from a GL and pharmaceutically acceptable salts thereof (hereinafter, these may be collectively abbreviated as GL (salts)); an oily base; water; and an alkali. The method for producing the GL-containing suppository composition for rectal infusion contains mixing the GL (salts), oily base, water, and alkali.

By containing the alkali, the GL-containing suppository composition for rectal infusion of the present aspect becomes soft, and can be prepared with ease. The reason for this is supposed to be that the existence of the alkali suppresses gelation effects of the GL (salts). Moreover, it is supposed that by suppressing the gelation effects of the GL, the dispersibility thereof in the rectum is enhanced, and thereby, the absorption of the GL can be enhanced.

The alkali is not particularly limited, provided that the solution thereof is basic, and examples thereof include compounds generally used as pH adjusters. Examples of such compounds include phosphates of alkali metal elements such as sodium, potassium, and the like, carbonates of alkali metal elements, hydroxides of alkali metal elements, hydroxides of alkaline earth metal elements such as calcium, and the like, amines such as triethanolamine, and the like, ammonia, and the like.

Examples of the phosphate of the alkali metal elements include disodium hydrogen phosphate ($Na_2HPO_4$), tripotassium phosphate ($K_3PO_4$), and the like.

Examples of the carbonates of the alkali metal elements include sodium carbonate ($Na_2CO_3$), sodium hydrogencarbonate ($NaHCO_3$), potassium carbonate ($K_2CO_3$), and the like.

Examples of the hydroxide of the alkali metal elements include sodium hydroxide, potassium hydroxide, and the like.

Among these, at least one selected from the group consisting of ammonia, phosphates, carbonates, and hydroxides of alkali metal elements is preferable, and the phosphates of alkali metal elements are particularly preferable, because the stability of the quality of the preparations becomes excellent.

The content of the alkali in the GL-containing suppository composition for rectal infusion may be suitably determined in accordance with the content of the GL (salts), the kind of the alkali to be used, or the like. For example, it is preferable that the content of the alkali be increased in accordance with the increase of the content of the GL (salts).

For example, when the content of the GL (salts) is 5 to 15% by mass of the preparation, the content of the alkali in the GL-containing suppository composition for rectal infusion is preferably 0.001 to 10.0% by mass, more preferably 0.01 to 7.5% by mass, and even more preferably 0.1 to 5.0% by mass, of the preparation. When the content of the alkali is no less than the lower limit of the above-mentioned range, effects of formulating the alkali can be sufficiently exhibited. In contrast, when the content is no more than the upper limit of the above-mentioned range, the stability of the quality of the preparation becomes excellent.

When the alkali is sodium hydroxide (NaOH) and/or potassium hydroxide (KOH), for example, the content of the alkali in the GL-containing suppository composition for rectal infusion is preferably 0.001 to 1.0% by mass.

When the alkali is monoethanolamine ($H_2NCH_2CH_2OH$), the content of the alkali in the GL-containing suppository composition for rectal infusion is preferably 0.002 to 1.0% by mass.

When the alkali is sodium carbonate ($Na_2CO_3$), the content of the alkali in the GL-containing suppository composition for rectal infusion is preferably 0.004 to 3.0% by mass.

When the alkali is disodium hydrogen phosphate ($Na_2HPO_4$), the content of the alkali in the GL-containing suppository composition for rectal infusion is preferably 0.005 to 7.5% by mass.

When the alkali is ammonia, the content of the alkali in the GL-containing suppository composition for rectal infusion is preferably 0.002 to 1.0% by mass.

The "hardness of the GL-containing suppository composition for rectal infusion" can be evaluated by determining the penetration-degree in accordance with the following procedure.

The GL-containing suppository composition for rectal infusion is filled into a sample vial (manufactured by AS ONE CORPORATION under the trade name of LABORAN screw tube No. 2; of which content is 6 cc; and bore diameter (inner diameter) is 10 mm) without containing air, and the measurement axis of a force gage (manufactured by A&D Co., LTD.) fixed at a motorized test stand (MODEL-2257 manufactured by Aikoh Engineering Corporation.) is inserted into the sample at the insertion rate of 60 mm/min. The load (of which the unit is newton (N)) is measured at the time of penetration as the penetration-degree. At this time, the measurement axis of the force gage is equipped with an elongated rod (of which length is 12 mm) and a compressive-disc type attachment (of which diameter is 8 mm).

In the present aspect, the penetration-degree determined by the above-mentioned measuring method is preferably 10.0 N or less, more preferably 5.0 N or less, and even more preferably 3.0 N or less. Although the lower limit of the penetration-degree is not particularly limited, the penetration-degree is preferably 0.01 N or more, and more preferably 0.1 N or more in view of the ease of handling the preparation.

As the GL and pharmaceutically acceptable salts thereof available in the present aspect, those available from Minophagen Pharmaceutical Co., LTD., can be exemplified. Examples of the pharmaceutically acceptable salts thereof include ammonium salts such as GL monoammonium salts and the like; sodium salts such as GL monosodium salts, GL disodium salts, and the like; potassium salts such as GL monopotassium salts, GL dipotassium salts, and the like. In addition to these, calcium salts, magnesium salts, aluminum salts of the GL, and various organic amine salts thereof can be exemplified. These can be used alone or in combination with at least two kinds thereof.

The content of the GL (salts) in the GL-containing suppository composition for rectal infusion is preferably 30% by mass or less, more preferably 1 to 20% by mass, even more preferably 1 to 15% by mass, and particularly preferably 5 to 15% by mass. When the content of the GL (salts) exceeds 30% by mass, it may become difficult to ensure uniform dispersion of the GL (salts) and the absorbability of the GL (salts) may decrease. When the content is 1% by mass or more, effects of the GL (salts) can be sufficiently exhibited. When the content is within the above-mentioned range, in the case of the preparation containing 10% by mass of the GL (salts), for example, the GL can be administered at the daily maximum dose of 200 mg by administering 2 g of the preparation, and thereby, the preparation can be practically used from the standpoint of clinical practice of the GL-containing rectal infusion suppository.

The oily base available in the present aspect is not particularly limited, and those generally used for a suppository can be used, such as, for example, medium chain fatty acid triglycerides, fatty acid glyceryl esters derived from vegetables such as soybean oil, olive oil, or the like.

As the medium chain fatty acid triglycerides, those containing fatty acids having 6 to 12 carbon atoms, such as caproic acid, caprylic acid, capric acid, dodecanoic acid, and the like, can be exemplified. Three medium chain fatty acids contained in one molecule of the medium chain fatty acid triglyceride may be identical to or different from each other.

As the medium chain fatty acid triglyceride, those of which fatty acid components contain 50 to 80% by mass of caprylic acid and 20 to 45% by mass of capric acid are particularly preferable. As such medium chain fatty acid triglycerides, one manufactured by SASOL CHEMICAL INDUSTRIES LTD under the trade name of "MIGLYOL 812" is commercially available, for example.

The content of the oily base in the GL-containing suppository composition for rectal infusion of the present aspect is preferably 10 to 50% by mass, and more preferably 10 to 40% by mass. When the content of the oily base is within the above-mentioned range, the stability of the quality of the preparation is high, and effects of the present aspect can be sufficiently exhibited.

As water available in the present aspect, purified water and the like can be exemplified.

The content of water in the GL-containing suppository composition for rectal infusion of the present aspect is preferably 20 to 70% by mass, and more preferably 30 to 60% by mass. When the content of water is outside the above-mentioned range, it may become difficult to ensure uniform dispersion of the GL in the GL-containing suppository composition for rectal infusion, and the absorbability of the GL (salts) may decrease.

The GL-containing suppository composition for rectal infusion of the present aspect may further contain a nonionic surfactant in addition to the above-mentioned components. In this case, the absorption of the GL (salts) from the rectum is increased, and the GL is transferred inside the body while remaining as a glycoside, as a result of which the content of the GL (salts) per suppository or the daily dose thereof is not required to be significantly increased, and thereby, high safety can be realized. The reason for increasing the rectal absorption of the GL (salts) by formulating the nonionic surfactant is supposed to be that the emulsifiability and dispersibility of the GL-containing suppository composition for rectal infusion in the rectum are improved.

Examples of the nonionic surfactant include sugar esters such as polyglycerol fatty acid esters, glyceryl fatty acid esters, sorbitan fatty acid esters (C12 to 18), polyoxyethylene (POE) sorbitan fatty acid esters (C12 to 18), sucrose fatty acid esters, and the like, fatty acid esters such as POE fatty acid esters (C12 to 18), POE resin acid esters, POE fatty acid diesters (C 12 to 18), and the like; alcohols such as POE alkyl ethers (C12 to 18), and the like; alkylphenols such as POE alkyl (C8 to 12) phenyl ethers, POE dialkyl (C8 to 12) phenyl ethers, POE alkyl (C8 to 12) phenyl ether formalin condensates, and the like; POE - POP block polymers such as POE - polyoxypropylene (POP) block polymers, alkyl (C12 to 18) POE - POP block polymer ethers, and the like; alkylamines such as POE alkylamine (C12 to 18), POE fatty acid amides (C12 to 18), and the like; vegetable oils such as POE castor oils, POE hardened castor oils, and the like.

Among these, at least one selected from the group consisting of sucrose fatty acid esters and polyglycerol fatty acid esters is preferable.

Examples of the sucrose fatty acid esters include those of which the fatty acid ester has 12 to 18 carbon atoms, such as laurate ester, palmitate ester, stearic acid ester, or the like, and of which ester components contain 55% or more of monoester component. Moreover, examples of the sucrose fatty acid esters commercially available include those manufactured by Mitsubishi-Kagaku Foods Corporation under the trade name of "J-1811 ", "J-1816", "J-1616", and "J-1216", and those manufactured by DAI-ICHI KOGYO SEIYAKU CO., LTD. under the trade name of "SS", "F-160", "F-110", "F-20W", "F-10", and the like.

As the polyglycerol fatty acid esters, those manufactured by Mitsubishi-Kagaku Foods Corporation under the trade name of "S-24D" and "L-7D", PGMSV manufatured by The Nisshin OilliO Group, Ltd., and the like, are commercially available.

As the nonionic surfactant, one kind thereof may be used alone, or at least two kinds thereof may be used in combination. When at least two kinds of the nonionic surfactant are used in combination, it is preferable that at least two kinds of nonionic surfactants having different hydrophilicities be used. For example, a hydrophilic nonionic surfactant and a lipophilic nonionic surfactant may be used in combination.

The content of the nonionic surfactant in the GL-containing suppository composition for rectal infusion is preferably 0.5 to 20% by mass, and more preferably 1 to 15% by mass. When the content of the nonionic surfactant is within the above-mentioned range, effects of formulating the nonionic surfactant are sufficiently exhibited. The reason for this is supposed to be that when the content of the nonionic surfactant is within the above-mentioned range, the emulsifiability and dispersibility of the GL-containing suppository composition for rectal infusion in the rectum is high.

It is preferable that the (solid-liquid) phase transition point of the GL-containing suppository composition for rectal infusion at 37°C be 10 to 4500 Pa, more preferably 10 to 2000 Pa, and even more preferably 15 to 1500 Pa. When the (solid-liquid) phase transition point at 37°C is within the above-mentioned range, the rectal absorption of the GL is enhanced, and sufficient effects can be exhibited even if the dose is less than that of the prior arts.

The absorbability of the GL (salts) in the GL-containing suppository composition for rectal infusion of the present aspect can be evaluated by measuring the amount of the GL (salts) excreted into the bile, and then determining the ratio thereof with respect to the dose thereof, that is, the collection rate (%) in the bile.

Specifically, the bile is collected as each sample in accordance with a conventional method, the amount of the GL (salts) in the sample is determined in accordance with a semimicro HPLC method, and the collection rate (%) in the bile is calculated from the amount of the GL (salts) in the bile in accordance with the following formula, to evaluate the absorbability.

$$\text{Collection rate (\%) in the bile} = [\text{the amount of the GL (salts) collected in the bile}] \times 100 \; / \; [\text{the amount of the GL (salts) administered}]$$

The GL-containing suppository composition for rectal infusion of the present aspect may further contain optional components in addition to the above-mentioned components, provided that the effects of the present aspect are not impaired. As the optional components, an antiseptic, various stabilizers, or the like, can be exemplified.

The GL-containing suppository composition for rectal infusion of the present aspect is a semisolid composition containing the above-mentioned components, such as a semisolid matter obtained by dissolving the GL (salts), oily base, and alkali into water to obtain an alkali solution, and then arbitrarily adding and mixing a nonionic surfactant. This is filled into a container such as a syringe, tube, or the like to use it as an infusion-type suppository.

Mixing can be conducted, for example, using a homogenizer, automatic mortar, or the like, by a technique generally used for preparing a semisolid preparation such as a cream.

As described above, the hardness of the GL-containing suppository composition for rectal infusion becomes soft by adding an alkali to the CL (salts), oily base, and water, and thereby, the industrial preparation thereof can be easily conducted. Accordingly, it is possible to formulate the GL (salts) at a high concentration, and for example, a preparation containing 10% or more of the GL can be prepared. For example, in the case of a preparation containing 10% of the GL, 200 mg, the daily maximum dose, of the GL (salts) can be administered by administering 2 g of the preparation, and thereby the preparation is practically available from the standpoint of the clinical practice.

Since such a suppository composition for rectal infusion is sufficiently soft and can be easily discharged from a container even after the composition is made into a preparation, it is thereby easily available. The reason for exhibiting such effects is supposed to be that the presence of the alkali suppresses the gelation effect of the GL (salts).

Thus, the GL-containing suppository composition for rectal infusion of the present aspect is easily available, and is not invasive, so the Q.O.L of the patient can be significantly improved.

When the GL-containing suppository composition for rectal infusion of the present aspect contains the nonionic surfactant, the absorption of the GL (salts) from the rectum is significantly enhanced. Accordingly, it is supposed that when 200 mg, the daily maximum dose, of the GL (salts) is administered using the above-mentioned preparation containing 10% of the GL, therapeutic effects comparable with those exhibited by intravenously administrating the same dose of the GL (salts) can be exhibited. Also, since the content of the GL in the GL-containing suppository composition for rectal infusion or the daily dose of the GL-containing suppository composition for rectal infusion can decrease, the preparation with high safety can be obtained.

Moreover, the GL-containing suppository composition for rectal infusion does not need to contain an absorption promoter which may stimulate the mucous membrane, such as a capric acid, and thereby, the composition is highly safe.

As described above, the GL-containing suppository composition for rectal infusion of the present aspect is industrially produced with ease, and easily used, and thereby the composition can be applied for the same diseases as those treated with a GL-containing injectable product currently used in clinical practice, such as, for example, hepatic dysfunction caused by acute or chronic hepatic disease, skin disease such as eczema, skin inflammation, urticarial rash, universal pruritus, drug eruption, toxic eruption, or the like, various allergies, SARS, AIDS, or the like.

The GL-containing suppository composition for rectal infusion of the present aspect is preferably used in a hepatic disease-therapeutic agent, skin disease-therapeutic agent, antiallergic agent, SARS therapeutic agent, or AIDS therapeutic agent. Particularly, since the composition exhibits excellent effects of decreasing transaminase, which is an indicator of hepatic disorder of an animal with experimentally-induced hepatic disorder, the composition is preferably used in the hepatic disease-therapeutic agent, and can be applied with great convenience for the patients with chronic hepatic disease requiring a long-term treatment, in comparison with conventional injectable products.

The hepatic disease-therapeutic agent of the present aspect contains the GL-containing suppository composition for rectal infusion. The hepatic disease-therapeutic agent containing the GL-containing suppository composition for rectal infusion is suitably prescribed by clinicians in accordance with the body weight or health conditions of the patient to be treated. For example, the dose thereof may be determined up to a ceiling of 200 mg of GL administered once or over several times per day.

The skin disease-therapeutic agent of the present aspect contains the GL-containing suppository composition for rectal infusion. The skin disease-therapeutic agent containing the GL-containing suppository composition for rectal infusion may be suitably prescribed by clinicians in accordance with the body weight or health conditions of the patient to be treated. For example, the dose thereof may be determined up to a ceiling of 200 mg of GL administered once or over several times per day.

The antiallergic agent of the present aspect contains the GL-containing suppository composition for rectal infusion. The antiallergic agent containing the GL-containing suppository composition for rectal infusion may be suitably prescribed by clinicians in accordance with the body weight or health conditions of the patient to be treated. For example, the dose thereof may be determined up to a ceiling of 200 mg of GL administered once or over several times per day.

The SARS therapeutic agent of the present aspect contains the GL-containing suppository composition for rectal infusion. The SARS therapeutic agent containing the GL-containing suppository composition for rectal infusion may be suitably prescribed by clinicians in accordance with the body weight or health conditions of the patient to be treated. For example, the dose thereof may be determined up to a ceiling of 200 mg of GL administered once or over several times per day.

The AIDS therapeutic agent of the present aspect contains the GL-containing suppository composition for rectal infusion. The AIDS therapeutic agent containing the GL-containing suppository composition for rectal infusion may be suitably prescribed by clinicians in accordance with the body weight or health conditions of the patient to be treated. For example, the dose thereof may be determined up to a ceiling of 200 mg of GL administered once or over several times per day.

In the following, the present invention will be explained in more detail showing examples, however, the present invention is not limited to these.

[Examples]

Examples 1 to 6 and Comparative Example 1

A monoammonium glycyrrhizinate and oily base were mixed in each amount (% by mass) shown in Table 1, and a nonionic surfactant was added to the mixture and mixed uniformly using a mortar. To the mixture, water was added and sufficiently kneaded to obtain a GL-containing suppository composition for rectal infusion.
Abbreviations shown in Table 1 mean the following.

GL-NH$_4$:   Monoammonium glycyrrhizinate
J-1811:   Sucrose fatty acid ester of which HLB is 11 (manufactured by Mitsubishi-Kagaku Foods Corporation)
S-24D:   Polyglycerol fatty acid ester of which HLB is 10 (manufactured by Mitsubishi-Kagaku Foods Corporation)
L-7D:   Polyglycerol fatty acid ester of which HLB is 17 (manufactured by Mitsubishi-Kagaku Foods Corporation)
PGMSV:   Polyglycerol fatty acid ester of which HLB is 14 (manufactured by The Nisshin OilliO Group, Ltd.)
MIGLYOL:   MIGLYOL 812 (medium chain fatty acid triglyceride of which fatty acid component contains 50 to 65% of a caprylic acid and 30 to 45% of a capric acid; manufactured by SASOL CHEMICAL INDUSTRIES LTD)

Table 1

| | Components (% by mass) | | | | Phase transition point (Pa) |
|---|---|---|---|---|---|
| | GL-NH$_4$ | Nonionic surfactant | Purified water | Oily base | |
| Example 1 | 13.5 | J-1811 (12.0) | 35.0 | MIGLYOL 812 (39.5) | 741.4 |
| Example 2 | 13.5 | J-1811 (6.0) | 41.0 | MIGLYOL 812 (39.5) | 1169.4 |
| Example 3 | 13.5 | J-1811 (3.0) | 44.0 | MIGLYOL 812 (39.5) | 1973.1 |
| Example 4 | 13.5 | S-24D (12.0) | 60.0 | MIGLYOL 812 (14.5) | 1312.5 |
| Example 5 | 13.5 | L-7D (12.0) | 50.0 | MIGLYOL 812 (24.5) | 81.4 |
| Example 6 | 13.5 | PGMSV (12.0) | 60.0 | MIGLYOL 812 (14.5) | 823.5 |
| Comparative Example 1 | 13.5 | | 45.0 | MIGLYOL 812 (41.5) | 4923.2 |

Test Example 1

The phase transition point of obtained GL-containing suppository compositions for rectal infusion was measured in accordance with the following procedure.
The viscoelasticity was measured, assuming an internal body temperature of 37°C, using an apparatus for measuring viscoelasticity (manufactured by Thermo Electron Corporation (Karlsruher) under the trade name of RheoStress RS600) with a parallel plate (20φ, 35φ, 60φ) or a cone plate (60φ, 1°), suitably selected as a measurement geometry, and the intersection of G' (storage elastic modulus) and G" (loss elastic modulus) was determined when the tangent δ was 1. Results thereof are also shown in Table 1.
As shown in Table 1, the phase transition points of the preparations of Examples 1 to 3, in which sucrose fatty acid esters were used as a nonionic surfactant, and the preparations of Examples 4 to 6, in which polyglycerol fatty acid esters were used, were lower than that of the preparation of Comparative Example 1, in which no nonionic surfactant was used. Also, in Examples 1 to 3, in which the same nonionic surfactant was used, the phase transition point decreased in accordance with an increase of the content of the nonionic surfactant.

Test Example 2 <Absorbability of GL (salts)>

With respect to the GL-containing suppository compositions for rectal infusion obtained in Examples 1 to 4 and Comparative Example 1, the collection rate (%) in the bile was determined in accordance with the following procedure, to evaluate the absorbability of the GL (salts) based on the rate. Results thereof are shown in Table 2.

n = 2 to 3

1. SD male rats (of which body weight was approximately 220 to 260 g, and age was 8 weeks) were starved for 24 hours before conducting the test.
2. The rats were anesthetized using ether and urethane.
3. The abdominal cavity of the rats was opened and insertion into the bile duct was carried out.
4. Each GL-containing suppository composition for rectal infusion was administered in the rectum using a tube for rats so as to administer the GL at a dose of 10 mg/kg.
5. After administration, the anal region was fastened using "ARONALPHA" (registered trademark) manufactured by TOAGOSEI CO., LTD.
6. The bile was collected at 30-minute intervals till 1 hour after administration and then collected at 1-hour intervals till 4 hours after administration.
7. The concentration of the GL in the bile was determined by a semimicro HPLC method.

Table 2.

|  | Example 1 | Example 2 | Example 3 | Example 4 | Comparative Example |
|---|---|---|---|---|---|
| Collection rate in the bile (%) | 88.7 | 78.9 | 32.2 | 42.5 | 5.3 |

As is apparent from Table 2, the collection rate in the bile of the preparations of Examples 1 to 3 in which J-1811, sucrose fatty acid ester, was used as the nonionic surfactant was improved in accordance with the increase of the content of the nonionic surfactant, that is, the decrease of the phase transition point. In view of this, it was supposed that the preparations had excellent absorbability of the GL.
Also, the preparation of Example 4, in which another nonionic surfactant was used, exhibited good absorbability.
In contrast, the preparation of Comparative Example 1, in which no nonionic surfactant was contained, the phase transition point thereof being high, exhibited an extremely low collection rate in the bile.

Test Example 3

<Evaluation with respect to medicinal effects of the preparation of Example 1>

The GL-containing suppository composition for rectal infusion prepared in Example 1 was evaluated in accordance with the following procedure with respect to effects of decreasing transaminase (ALT), which is an indicator of hepatic inflammation, using a mouse with hepatic inflammation induced by lipopolysaccharide (LPS) / D(+)-galactosamine (GalN) in accordance with a method disclosed in Nonpatent Document 7.
[Nonpatent Document 7] Proc. Japan Acad., 79, Ser. B, p. 170 to 175 (2003)
The GL-containing suppository composition for rectal infusion was administered into the rectum of the mouse so that the dose of the GL was 100 mg/kg. After 30 minutes, a mixed solution of LPS and GalN (LPS / GalN) was intravenously injected to the tail of the mouse. After 8 hours, the blood was collected, and ALT activity was measured. Results thereof are shown in the column "Example 1 administration into the rectum" of Table 3.
As an untreated group, the GL-containing suppository composition for rectal infusion was not administered and saline was intravenously injected to the tail of the mouse instead of LPS/GalN. The remaining process was conducted as described above, and the ALT activity was measured. Results thereof are shown in the column "Untreated" of Table 3.
As a LPS/GalN group, the GL-containing suppository composition for rectal infusion was not administered and only LPS/GalN was intravenously injected to the tail of the mouse. The remaining process was conducted as described above, and the ALT activity was measured. Results thereof are shown in the column "LPS/GalN" of Table 3.
As a group of GL administration into the abdominal cavity, a solution of $GL-NH_4$ was administered inside the abdominal cavity so that the dose of the GL was 100 mg/kg. After 30 minutes, LPS/GalN was intravenously injected to the tail of the mouse as described above, and the ALT activity was measured. Nonpatent Document 8 has reported that such administration of the GL into the abdominal cavity exhibits the GL-absorption at the same level as that exhibited by intraveneous injection.

[Nonpatent Document 8] Biol. Pharm. Bull., 18(2), p337-341 (1995)

Table 3.

| | Untreated | LPS/GalN | GL administration into the abdominal cavity | Example 1 administration into the rectum |
|---|---|---|---|---|
| Administered medicine | Saline | LPS/GalN | LPS/GalN GL solution | LPS/GalN Example 1 |
| Transaminase (ALT) IU/L (Average) | 22.8 | 1183.5 | 289.2 * | 454.0* |
| *: p<0.05 with respect to LPS/GalN | | | | |

Test Example 4

<Evaluation with respect to medicinal effects of the preparation of Example 4>

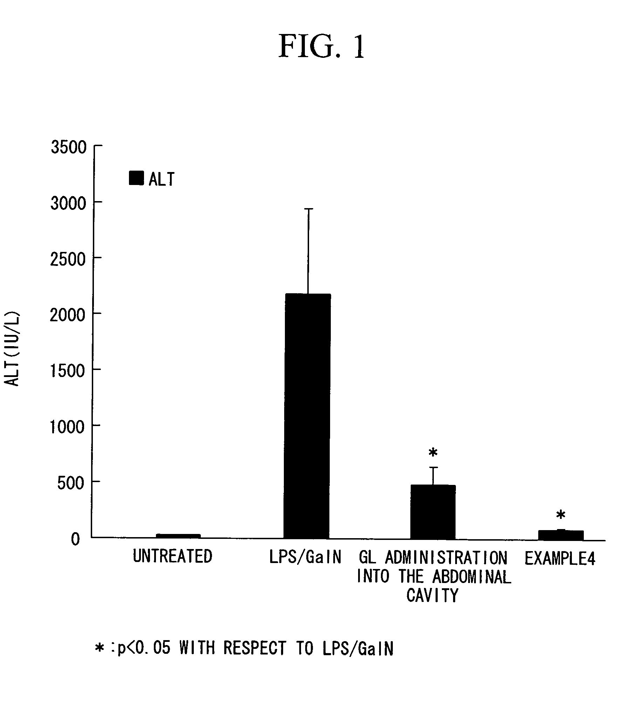

With respect to the GL-containing suppository composition for rectal infusion prepared in Example 4, evaluation was conducted in a similar manner to that of Test Example 3. Results thereof are shown in Table 4 and FIG. 1.

Table 4

| | Untreated | LPS/GalN | GL administration into the abdominal cavity | Example 4 administration into the rectum |
|---|---|---|---|---|
| Administered medicine | Saline | LPS/GalN | LPS/GalN GL solution | LPS/GalN Example 4 |
| Transaminase (ALT) IU/L (Average) | 25.8 | 2187.1 | 483.2* | 83.7 * |
| *: p<0.05 with respect to LPS/GalN | | | | |

In view of the results of Test Examples 3 and 4, the GL-containing suppository compositions for rectal infusion prepared in Examples 1 and 4 exhibited significant effects of decreasing transaminase at the same level as that exhibited by the administration of the GL into the abdominal cavity, and thereby the compositions are considered to be useful from the standpoint of the clinical practice.

Comparative Example 2 and Examples 7 to 13

Each GL-containing suppository composition for rectal infusion of which components and each content (% by mass) are shown in Table 5 was prepared by uniformly suspending and sufficiently kneading monoammonium glycyr-rhizinate (GL-NH$_4$), oily base, and either purified water or an alkali solution, and a nonionic surfactant if further formulated, using a homomixer.

With respect to the compositions of Examples 7 to 13 in which an alkali solution was used, the ratios (% by mass) of the water component and the alkali component are shown in parenthesis.

Abbreviations shown in Table 5 mean the following.

MIGLYOL: MIGLYOL 812 (medium chain fatty acid triglyceride of which fatty acid component contains 50 to 65% of a caprylic acid and 30 to 45% of a capric acid; manufactured by SASOL CHEMICAL INDUSTRIES LTD.)

Alkali solution A: 0.05 M Na$_2$HPO$_4$ solution
Alkali solution B: 0.1 M Na$_2$HPO$_4$ solution
Alkali solution C: 0.5 M Na$_2$HPO$_4$ solution
Alkali solution D: 1.0 M Na$_2$HPO$_4$ solution

Alkali solution E:    0.3 M NaOH solution
J-1811:               Sucrose fatty acid ester of which HLB is 11 (manufactured by Mitsubishi-Kagaku Foods Corporation)
L-7D:                 Polyglycerol fatty acid ester of which HLB is 17 (manufactured by Mitsubishi-Kagaku Foods Corporation)

Table 5

| | | Comparative Example 2 | Example 7 | Example 8 | Example 9 | Example 10 | Example 11 | Example 12 | Example 13 |
|---|---|---|---|---|---|---|---|---|---|
| GL-NH$_4$ | | 13.5 | 13.5 | 13.5 | 13.5 | 13.5 | 13.5 | 13.5 | 13.5 |
| Oily base/ MIGLYOL | | 41.5 | 41.5 | 39.5 | 39.5 | 39.5 | 39.5 | 39.5 | 39.5 |
| Purified water | | 45.0 | - (44.361) | - (34.752) | - (34.503) | - (32.516) | - (30.031) | - (34.580) | -(34.503) |
| Alkali solution | A | - | - | 35.0 (0.248) | - | - | - | - | - |
| | B | - | 45.0 (0.639) | - | 35.0 (0.497) | - | - | - | 35.0 (0.497) |
| | C | - | - | - | - | 35.0 (2.484) | - | - | - |
| | D | - | - | - | - | - | 35.0 (4.969) | - | - |
| | E | - | - | - | - | - | - | 35.0 (0.420) | - |
| Nonionic surfactant | J-1811 1 | - | - | 12.0 | 12.0 | 12.0 | 12.0 | 12.0 | - |
| | L-7D | -- | -- | -- | -- | -- | -- | -- | 12.0 |
| Total (% by mass) | | 100.0 | 100.0 | 100.0 | 100.0 | 100.0 | 100.0 | 100.0 | 100.0 |

Test Example 5

<Hardness of the GL-containing suppository composition for rectal infusion>

With respect to the GL-containing suppository compositions for rectal infusion prepared in Comparative Example 2 and Examples 7 to 13, the physical property of each preparation was evaluated by the penetration-degree using a force gage (manufactured by A&D Co., LTD.) fixed at a motorized test stand (manufactured by Aikoh Engineering Corporation., under the trade name of MODEL-2257).

The preparation to be evaluated was filled into a sample vial (manufactured by AS ONE CORPORATION under the trade name of LABORAN screw tube No. 2; of which content is 6 cc; and bore diameter (inner diameter) is 10 mm) without containing air, and the measurement axis of the force gage fixed at the motorized test stand was inserted into the sample. The load measured at the time of penetration was represented by newton (N) as the penetration-degree. At this time, the measurement axis of the force gage was equipped with an elongated rod (of which length was 12 mm) and a compressive-disc type attachment (of which diameter was 8 mm). The rate of inserting the force gage was set at 60 mm/min.

Results thereof are shown in Table 6. As is apparent from the results, the penetration-degree of the GL-containing suppository compositions for rectal infusion prepared in Examples 7 to 13 was lower, that is, the hardness thereof was softer, than that of the GL-containing suppository composition for rectal infusion prepared in Comparative Example 2. Specifically, the penetration-degree of the compositions prepared in Examples 8 to 13, in which nonionic surfactants were added, was low. The penetration-degree decreased in accordance with an increase in the content of the alkali added.

Table 6

| | Comparative Example 2 | Example 7 | Example 8 | Example 9 | Example 10 | Example 11 | Example 12 | Example 13 |
|---|---|---|---|---|---|---|---|---|
| Penetration-degree (N) | 10.15 | 4.54 | 2.98 | 0.74 | 0.46 | 0.24 | 0.63 | 0.91 |

Test Example 6

<Absorbability of GL (salts)>

With respect to the GL-containing suppository compositions for rectal infusion prepared in Comparative Example 2 and Examples 7 to 13, the absorbability of the GL (salts) was evaluated by determining the collection rate (%) in the bile in accordance with the following procedure. Results thereof are shown in Table 7.
n=2 to 5

1. SD male rats (of which body weight was approximately 220 to 260 g, and age was 8 weeks) were starved for 24 hours before conducting test.
2. The rats were anesthetized using ether and urethane.
3. The abdominal cavity of the rats was opened and insertion into the bile duct was carried out.
4. Each GL-containing suppository composition for rectal infusion was administered in the rectum using a tube for rats so as to administer the GL at a dose of 10 mg/kg.
5. After administration, the anal region was fastened using "ARONALPHA" (manufactured by TOAGOSEI CO., LTD).
6. The bile was collected at 30-minute intervals till 1 hour after administration and then collected at 1-hour intervals till 4 hours after administration.
7. The concentration of the GL in the bile was determined by a semimicro HPLC method.

Table 7

|  | Comparative Example 2 | Example 7 | Example 8 | Example 9 | Example 10 | Example 11 | Example 12 | Example 13 |
|---|---|---|---|---|---|---|---|---|
| Collection rate in the bile (%) | 5.3 | 6.8 | 75.9 | 90.0 | 79.6 | 72.6 | 99.6 | 17.9 |

As is apparent from the results in Table 7, the collection rate in the bile of the compositions prepared in Example 8 to 13, in which nonionic surfactants were added, was significantly increased. In view of this, it is supposed that the compositions exhibit a high rate of GL-absorption in clinical practice.

Test Example 7

<Evaluation of medicinal effects of the preparations prepared in Examples 8 and 9 >

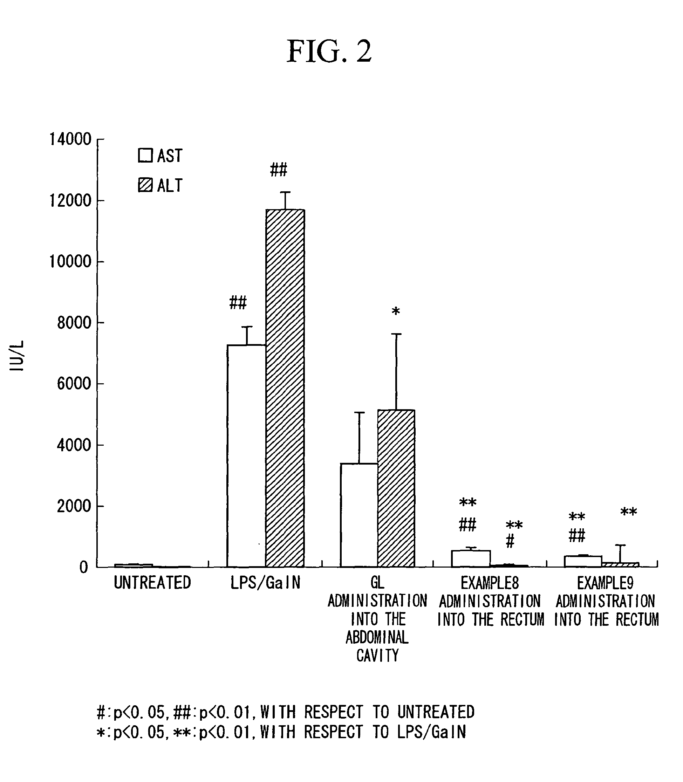

The GL-containing suppository compositions for rectal infusion prepared in Examples 8 and 9 were evaluated in accordance with the following procedure with respect to effects of decreasing transaminase activity (ALT (alanine aminotransferase) activity) and AST (aspartate aminotransferase) activity, which are indicators of hepatic inflammation, using a mouse with hepatic inflammation induced by lipopolysaccharide (LPS) / D(+)-galactosamine (GalN) in accordance with a method disclosed in Nonpatent Document 8.
The GL-containing suppository compositions for rectal infusion prepared in Examples 8 and 9 were administered into the rectum of the mouse so that the dose of the GL was 100 mg/kg. After 30 minutes, a mixed solution of LPS and GalN (LPS / GalN) was intravenously injected to the tail of the mouse. After 8 hours, the blood was collected, and the ALT activity was measured. Results thereof are shown in the column "Example 8 administration into the rectum" and "Example 9 administration into the rectum" of Table 8 and FIG. 2.
As an untreated group, no GL-containing suppository composition for rectal infusion was administered, but saline was intravenously injected to the tail of the mouse instead of LPS/GalN. The remaining process was conducted as described above, and the transaminase activity was measured. Results thereof are shown in the column "Untreated" of Table 8 and FIG. 2.
As a LPS/GalN group, the GL-containing suppository composition for rectal infusion was not administered and only LPS/GalN was intravenously injected to the tail of the mouse. The remaining process was conducted as described above, and the transaminase activity was measured. Results thereof are shown in the column "LPS/GalN" of Table 8 and FIG. 2.
As a group of GL administration into the abdominal cavity, a solution of $GL-NH_4$ was administered inside the abdominal cavity so that the dose of the GL was 100 mg/kg. After 30 minutes, LPS/GalN was intravenously injected to the tail of the mouse as described above, and the transaminase activity was measured. Results thereof are shown in the column "GL administration into the abdominal cavity" of Table 8 and FIG. 2. Nonpatent Document 7 has reported that such administration of the GL into the abdominal cavity exhibits the GL-absorption at the same level as that exhibited by intraveneous injection.

Table 8

|  | Untreated | | LPS/GalN | | GL administration into the abdominal cavity | | Example 8 administration into the rectum | | Example 9 administration into the rectum | |
|---|---|---|---|---|---|---|---|---|---|---|
| Transaminase activity (Average) (IU/L) | ALT | AST | ALT | AST | ALT | AST | ALT | AST | ALT | AST |
|  | 13 | 78 | 11700 | 7258 | 5129* | 3374 | 47** | 534** | 125** | 353** |
| *: $p < 0.05$, **: $p < 0.01$, with respect to LPS/GalN | | | | | | | | | | |

As a result of Test Example 7, the GL-containing suppository compositions for rectal infusion prepared in Examples 8 and 9 exhibited significant effects of decreasing transaminase activity, and thereby, the compositions were supposed to be useful from the standpoint of clinical practice.

Examples 14 to 27

Each GL-containing suppository composition for rectal infusion of which components and each content (% by mass) are shown in Tables 9 to 11 was prepared by uniformly suspending and sufficiently kneading monoammonium glycyrrhizinate (GL-NH$_4$), oily base, nonionic surfactant and either purified water or an alkali solution, using a homomixer. In Example 25, a benzalkonium chloride was further added to the mixture to prepare a composition.

With respect to the compositions of Examples 19 to 27 in which an alkali solution was used, the ratios (% by mass) of the water component and the alkali component are shown in parenthesis.

Abbreviations shown in Tables 9 to 11 mean the following. SS: Sucrose fatty acid esters (manufactured by DAI-ICHI KOGYO SEIYAKU CO., LTD.) of which HLB is 19.

F-160: Sucrose fatty acid esters (manufactured by DAI-ICHI KOGYO SEIYAKU CO., LTD.) of which HLB is 15.

J-1811: Sucrose fatty acid esters (manufactured by Mitsubishi-Kagaku Foods Corporation) of which HLB is 11.

F-20W: Sucrose fatty acid esters (manufactured by DAI-ICHI KOGYO SEIYAKU CO., LTD.) of which HLB is 2.

F-10: Sucrose fatty acid esters (manufactured by DAI-ICHI KOGYO SEIYAKU CO., LTD.) of which HLB is 1.

MIGLYOL: MIGLYOL 812 (medium chain fatty acid triglyceride of which fatty acid component contains 50 to 65% of a caprylic acid and 30 to 45% of a capric acid; manufactured by SASOL CHEMICAL INDUSTRIES LTD.)

With respect to the prepared GL-containing suppositories for rectal infusion, the phase transition point thereof was measured in accordance with the method used in Test Example 1, and the penetration-degree thereof was measured in accordance with the method used in Test Example 5. Also, the collection rate in the bile was measured in accordance with the method used in Test Example 6. Results thereof are shown in Tables 12 and 13.

Table 9

|  | Example 14 | Example 15 | Example 16 | Example 17 | Example 18 |
|---|---|---|---|---|---|
| GL-NH$_4$ | 13.5 | 13.5 | 13.5 | 13.5 | 13.5 |
| SS | - | - | 6.0 | 12.0 | - |
| J-1811 | 2.0 | 12.0 | 4.0 | - | 12.0 |
| F-10 | - | - | 2.0 | - | - |
| Purified water | 45.0 | 40.0 | 35.0 | 35.0 | 64.5 |
| MIGLYOL | 39.5 | 34.5 | 39.5 | 39.5 | 10.0 |
| Total (% by mass) | 100.0 | 100.0 | 100.0 | 100.0 | 100.0 |

(continued)

|  | Example 14 | Example 15 | Example 16 | Example 17 | Example 18 |
|---|---|---|---|---|---|
| Phase transition point (Pa) | 1202.3 | 57.00 | 107.62 | 215.39 | 362.50 |
| Penetration-degree | 5.6 | 0.44 | 0.06 | 0.02 | 0.58 |

Table 10

|  | Example 19 | Example 20 | Example 21 | Example 22 | Example 23 | Example 24 | Example 25 | Example 26 |
|---|---|---|---|---|---|---|---|---|
| GL-NH$_4$ | 13.5 | 13.5 | 13.5 | 13.5 | 13.5 | 13.5 | 13.5 | 13.5 |
| F-160 | - | - | - | - | - | 6.0 | - | - |
| J-1811 | 12.0 | 12.0 | 12.0 | 12.0 | 12.0 | - | 12.0 | 12.0 |
| F-110 | - | - | - | - | - | 4.0 | - | - |
| F-20W | - | - | - | - | - | 2.0 | - | - |
| Purified water | - (59.148) | - (57.177) | - (39.432) | - (27.547) | 31 (34.6) | - (34.503) | 30.97 (34.57) | -(49.290) |
| 10% Ammonia water | - | - | - | - | 4 (0.4) | - | 4 (0.4) | - |
| 0.1M Na$_2$HPO$_4$ solution | 60 (0.852) | 58 (0.823) | 40 (0.568) | - | - | 35 (0.497) | - | 50 (0.710) |
| 1.5M Na$_2$HPO$_4$ solution | - | - | - | 35 (7.453) | - | - | - | - |
| MIGLYOL | 14.5 | 16.5 | 34.5 | 39.5 | 39.5 | 39.5 | 39.5 | 24.5 |
| Benzalkonium chloride | - | - | - | - | - | - | 0.03 | - |
| Total (% by mass) | 100.0 | 100.0 | 100.0 | 100.0 | 100.0 | 100.0 | 100.0 | 100.0 |
| Phase transition point (Pa) | 15.49 | 245.63 | 51.00 | 69.32 | 273.18 | 773.37 | 539.47 | 177.02 |
| Penetration-degree (N) | 0.01 | 0.02 | 0.15 | 0.03 | 0.04 | 0.73 | 0.03 | 0.10 |

Table 11

|  | Example 27 |
|---|---|
| GL-NH$_4$ | 27.0 |
| F-110 | 10.0 |
| Purified water | 37 (42.4) |
| 10% Ammonia water | 6.0 (0.6) |
| MIGLYOL | 20.0 |
| Total (% by mass) | 100.0 |

Table 12

|  | Example 14 | Example 15 | Example 16 |
|---|---|---|---|
| Collection rate in the bile (%) | 35.86 | 69.4 | 71.08 |

Table 13

|  | Example 19 | Example 20 | Example 21 | Example 22 | Example 23 | Example 24 | Example 27 |
|---|---|---|---|---|---|---|---|
| Collection rate in the bile (%) | 66.68 | 56.88 | 77.86 | 37.09 | 60.13 | 83.06 | 30.54 |

As shown in Tables 12 and 13, the GL-containing suppository compositions for rectal infusion prepared in the examples also exhibited a high collection rate in the bile, and thereby, it was supposed that the compositions exhibit excellent GL-absorbability in clinical practice.

INDUSTRIAL APPLICABILITY

The present invention provides a GL-containing suppository composition for rectal infusion which can be industrially produced with ease, and exhibits an excellent GL-absorbability, and a production method thereof, and a hepatic disease-therapeutic agent, skin disease-therapeutic agent, antiallergic agent, SARS therapeutic agent, and AIDS therapeutic agent containing the GL-containing suppository composition for rectal infusion.

Moreover, the GL-containing suppository composition for rectal infusion according to the present invention can contain the GL at a high concentration without deteriorating the absorbability of the GL. Accordingly, in the case of a preparation containing 10% of the GL, for example, by administrating 2g of the preparation, the daily maximum dose, 200 mg, of the GL can be administered, and thereby, the preparation is practically available from the standpoint of clinical practice, and probably exhibits therapeutic effects at the same level as those ejected by intravenous administration thereof.

The suppository composition for rectal infusion according to the present invention is soft and this softness is maintained after the composition is made into a preparation, and thereby, the composition is easily exhausted from a container, and so is easily used.

Thus, the GL-containing suppository composition for rectal infusion according to the present invention is easily used and is not invasive, and thereby, can significantly improve the Q.O.L of the patients.

Moreover, when the GL-containing suppository composition for rectal infusion according to the present invention contains a nonionic surfactant, the absorption of the GL (salts) from the rectum is significantly improved, and the GL is transferred inside the body while remaining as a glycoside, high therapeutic effects comparable with those exhibited by intravenous administration can be exhibited. Accordingly, the GL-containing suppository composition for rectal infusion is not required to contain the GL in an amount significantly exceeding the daily maximum dose thereof, and thereby, is highly safe.

Moreover, the GL-containing suppository composition for rectal infusion is not required to contain an absorption promoter such as a capric acid, which may stimulate the mucous membrane, and thereby, is highly safe.

Since the GL-containing suppository composition for rectal infusion according to the present invention is industrially produced with ease and is easily used, the composition can be applied for the same diseases as those treated with GL-containing injectable products currently used in clinical practice, such as hepatic dysfunction caused by acute or chronic hepatic disease, skin disease such as eczema, skin inflammation, urticarial rash, universal pruritus, drug eruption, toxic eruption, or the like, various allergies, SARS, AIDS, or the like.

Particularly, the GL-containing suppository composition for rectal infusion according to the present invention can be preferably used in a hepatic disease-therapeutic agent, skin disease-therapeutic agent, antiallergic agent, SARS therapeutic agent, or AIDS therapeutic agent. Among these, the composition can be preferably used in the hepatic disease-therapeutic agent, and can be applied for patients with chronic hepatic disease requiring a long-term treatment with great convenience in comparison with conventional injectable products.

The suppository containing the GL-containing suppository composition for rectal infusion provides the treatment with low cost in comparison with the treatment using IFN, and thereby, is useful from the standpoint of medical economics.

**Claims**

1. A glycyrrhizin-containing suppository composition for rectal infusion **characterized by** comprising: at least one selected from a glycyrrhizin and pharmaceutically acceptable salts thereof; a nonionic surfactant; an oily base; and water, wherein
a (solid-liquid) phase transition point of the glycyrrhizin-containing suppository composition for rectal infusion at 37°C is within a range from 10 to 4500 Pa.

2. The glycyrrhizin-containing suppository composition for rectal infusion according to Claim 1, **characterized in that** the nonionic surfactant is at least one selected from the group consisting of sucrose fatty acid esters and polyglycerol fatty acid esters.

3. A hepatic disease-therapeutic agent comprising the glycyrrhizin-containing suppository composition for rectal infusion of Claim 1 or 2.

4. A skin disease-therapeutic agent comprising the glycyrrhizin-containing suppository composition for rectal infusion of Claim 1 or 2.

5. An antiallergic agent comprising the glycyrrhizin-containing suppository composition for rectal infusion of Claim 1 or 2.

6. A SARS therapeutic agent comprising the glycyrrhizin-containing suppository composition for rectal infusion of Claim 1 or 2.

7. An AIDS therapeutic agent comprising the glycyrrhizin-containing suppository composition for rectal infusion of Claim 1 or 2.

8. A glycyrrhizin-containing suppository composition for rectal infusion
**characterized by** comprising: at least one selected from a glycyrrhizin and pharmaceutically acceptable salts thereof; an oily base; water; and an alkali.

9. The glycyrrhizin-containing suppository composition for rectal infusion according to Claim 8, **characterized in that** the alkali is at least one selected from the group consisting of alkali metal phosphates, alkali metal carbonates, alkali metal hydroxides, alkaline earth metal hydroxides, amines, and an ammonia.

10. The glycyrrhizin-containing suppository composition for rectal infusion according to Claim 8 or 9, **characterized by** further comprising a nonionic surfactant.

11. The glycyrrhizin-containing suppository composition for rectal infusion according to Claim 10, **characterized in that** the nonionic surfactant is at least one selected from the group consisting of sucrose fatty acid esters and polyglycerol fatty acid esters.

12. A method for producing the glycyrrhizin-containing suppository composition for rectal infusion of any one of Claims 8 to 11, **characterized by** comprising mixing at least one selected from a glycyrrhizin and pharmaceutically acceptable salts thereof, an oily base, water, and an alkali.

13. A hepatic disease-therapeutic agent comprising the glycyrrhizin-containing suppository composition for rectal infusion of any one of Claims 8 to 11.

14. A skin disease-therapeutic agent comprising the glycyrrhizin-containing suppository composition for rectal infusion of any one of Claims 8 to 11.

15. An antiallergic agent comprising the glycyrrhizin-containing suppository composition for rectal infusion of any one of Claims 8 to 11.

16. A SARS therapeutic agent comprising the glycyrrhizin-containing suppository composition for rectal infusion of any one of Claims 8 to 11.

17. An AIDS therapeutic agent comprising the glycyrrhizin-containing suppository composition for rectal infusion of any one of Claims 8 to 11.

# FIG. 1

* :p<0. 05 WITH RESPECT TO LPS/GaIN

# FIG. 2

#:p<0. 05, ##:p<0. 01, WITH RESPECT TO UNTREATED
*:p<0. 05, **:p<0. 01, WITH RESPECT TO LPS/GaIN

<div align="center">

**INTERNATIONAL SEARCH REPORT**

</div>

| International application No. |
| --- |
| PCT/JP2005/011958 |

A. CLASSIFICATION OF SUBJECT MATTER
Int.Cl⁷ A61K31/704, 9/02, 47/02, 47/14, 47/18, 47/26, 47/34, 47/44,
        A61P1/06, 17/00, 37/08

According to International Patent Classification (IPC) or to both national classification and IPC

B. FIELDS SEARCHED

Minimum documentation searched (classification system followed by classification symbols)
Int.Cl⁷ A61K31/704, 9/02, 47/02, 47/14, 47/18, 47/26, 47/34, 47/44

Documentation searched other than minimum documentation to the extent that such documents are included in the fields searched
Jitsuyo Shinan Koho          1922-1996   Jitsuyo Shinan Toroku Koho   1996-2005
Kokai Jitsuyo Shinan Koho    1971-2005   Toroku Jitsuyo Shinan Koho   1994-2005

Electronic data base consulted during the international search (name of data base and, where practicable, search terms used)
REGISTRY(STN), CAPLUS(STN)

C. DOCUMENTS CONSIDERED TO BE RELEVANT

| Category* | Citation of document, with indication, where appropriate, of the relevant passages | Relevant to claim No. |
| --- | --- | --- |
| X<br>Y | WO 2004/047846 A1 (Kabushiki Kaisha Minofagen Seiyaku),<br>10 June, 2004 (10.06.04),<br>Full text; particularly, Claims; page 1, lines 9 to 13; page 8, lines 19 to 24; examples; test examples 8 to 9<br>(Family: none) | 1-7<br>8-17 |
| Y | JP 5-097680 A (Sanwa Kagaku Kenkyusho Co., Ltd.),<br>20 April, 1993 (20.04.93),<br>Full text; particularly, Claims; Par. Nos. [0007], [0008], [0012]; examples<br>& EP 535704 A1 | 1-17 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>11 August, 2005 (11.08.05) | Date of mailing of the international search report<br>30 August, 2005 (30.08.05) |
| --- | --- |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

| International application No. |
| --- |
| PCT/JP2005/011958 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 4-261117 A  (Santen Pharmaceutical Co., Ltd.), 17 September, 1992 (17.09.92), Full text; particularly, Claims; examples (Family: none) | 1-17 |
| Y | JP 7-082155 A  (Minophagen Pharmaceutical Co.), 28 March, 1995 (28.03.95), Full text; particularly, Claims; Par. Nos. [0014], [0017], [0020]; examples & JP 3534793 B | 1-17 |
| Y | JP 5-032521 A  (MAXFACTOR Kabushiki Kaisha), 09 February, 1993 (09.02.93), Full text; particularly, Par. No. [0002] & GB 2257907 A        & AU 9220530 A & CN 1068731 A | 1-17 |
| Y | JP 6-305932 A  (Maruzen Pharmaceuticals Co., Ltd.), 01 November, 1994 (01.11.94), Full text; particularly, Par. No. [0003] (Family: none) | 1-17 |
| Y | JP 58-194805 A  (Shiseido Co., Ltd.), 12 November, 1983 (12.11.83), Full text; particularly, page 1, right column, lines 14 to 20 (Family: none) | 1-17 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004191275 A
- JP 2004371222 A
- JP H32122 A
- JP H10226650 A
- JP H6192107 B

**Non-patent literature cited in the description**

- *THE LANCET,* 2003, vol. 361 (934), 2045-2046
- *Antiviral Research,* 1989, vol. 11, 255-262
- *Hepatol. Res.,* 2003, vol. 26, 10-14
- *Chem. Pharm. Bull.,* 1990, vol. 38 (1), 212-218
- *J. Pharm. Sci.,* 1986, vol. 75 (7), 672-675
- *Pharmaceutical Society of Japan,* 11-3
- *Proc. Japan Acad.,* 2003, vol. 79, 170-175
- *Biol. Pharm. Bull.,* 1995, vol. 18 (2), 337-341